# EUROPEAN PATENT SPECIFICATION

(11) **EP 2 051 649 B1**
(45) Date of publication and mention of the grant of the patent: **25.04.2012**
(21) Application number: 07805288.3
(22) Date of filing: 02.08.2007
(51) Int. Cl.: A61B 18/12, A61B 19/00, A61B 18/14

(54) **IMAGE-BASED POWER FEEDBACK FOR OPTIMAL ULTRASOUND IMAGING OF RADIO FREQUENCY TISSUE ABLATION**
BILDBASIERTE LEISTUNGSRÜCKMELDUNG FÜR DIE OPTIMALE ULTRASCHALLBILDGEBUNG EINER FUNKFREQUENZ-GEWEBEABLATION
RETROACTION DE PUISSANCE REALISEE A PARTIR D'UNE IMAGE D'UNE ABLATION DE TISSU PAR RADIOFREQUENCES POUR IMAGERIE PAR ULTRASONS AMELIOREE

(30) Priority: 11.08.2006 US 822125 P
(43) Date of publication of application: 29.04.2009
(73) Proprietor: Koninklijke Philips Electronics N.V., 5621 BA Eindhoven (NL)
(72) Inventor: SAVERY, David, Briarcliff Manor, New York 10510-8001 (US); HALL, Christopher, Briarcliff Manor, New York 10510-8001 (US)
(74) Representative: Schouten, Marcus Maria
(86) International application number: PCT/IB2007/053047
(87) International publication number: WO 2008/017990

(56) References cited:
- WO-A-2006/064495
- GB-A- 2 187 840
- US-A- 5 657 760
- US-A- 5 694 936
- US-A1- 2004 267 120
- US-A1- 2005 283 074

## Description

The technical field of the invention is providing a system for optimizing ultrasound images during radiofrequency (RF) ablation by providing feedback from real-time imaging and controlling RF power.

RF ablation is a curative, clinical procedure often used for tumor destruction in treating diverse classes of cancer, such as hepatic metastases or hepatocellular carcinoma. RF ablation is a promising procedure for treating cancer patients who cannot undergo resection surgery. The clinical objective of RF ablation is to thermally ablate cancerous tissue while sparing healthy parenchyma to ensure that side effects of treatment are minimal.

RF ablation is a minimally invasive procedure that needs to be guided and monitored by an external interventional imaging modality. Currently, imaging modalities that are most commonly used for guidance and monitoring of RF ablation are ultrasound and computed tomography. As ultrasound scans provide real-time images, with virtually no harmful radiation and at a relatively moderate cost, this technique has great existing and untapped promise for guidance and monitoring of interventional procedures. Advantages of ultrasound include its real time capabilities and cost aspects, however due negative impacts of cavitation resulting from intensity of heating during RF treatment, ultrasound image quality can be diminshed.

During RF ablation treatment, body temperature is increased locally at levels to induce necrosis, i.e. death of cells or tissue, in a targeted area. An RF probe is inserted into the target tissue, usually percutaneously. Heat is produced by dielectric loss, at the passage of a RF current generated at the tip(s) of the probe. During heating, the temperature of the tissue surrounding the tip of the probe can reach the boiling point (close to 90-100 °C), which results in cavitation, i.e. the formation of bubble pockets. The presence of bubbles affects the propagation of an acoustic imaging wave through the medium, and disrupts ultrasound image quality. When bubbles are present, the efficacy of ultrasonic monitoring of the procedure is readily degraded by the "shadowing" or loss of signal distal to a gas pocket. Furthermore, generation of bubbles can also modify the outcome of the treatment itself, since air is a good insulator and can therefore prevent heat diffusion within tissue. It is therefore desirable to prevent bubble formation to improve visualization of the RF ablation procedure on the ultrasound scanner.

Accordingly, an object of the present invention is optimizing ultrasound images by controlling RF power to minimize the formation of bubbles, while at the same maximizing the efficacy of RF ablation therapy. Using the ultrasound data supplied by an ultrasound imaging system as feedback parameters, the RF power generated during RF ablation treatment is limited in order to avoid heat-induced cavitation.

The document US-A1-2005283074 discloses a system comprising an ultrasound scanner configured to acquire at least one image of a target area for use during a radiofrequency (RF) ablation therapy, a radiofrequency (RF) probe configured to be inserted into the target area, an RF power generator and a bubble detector configured to indicate a presence of at least one bubble in the target area and produce a feedback signal, wherein the RF power generator is configured to be altered in response to the feedback signal.

The invention is set out in claim1, which defines a system that includes among other features an ultrasound scanner that acquires a pre-treatment image of a target area for calibration, and at least one additional image of the target area; a radiofrequency (RF) probe, such that the RF probe is inserted into the target area; an RF power generator; and a bubble detector, such that the bubble detector indicates a presence of at least one bubble in the target area and produces a feedback signal, and such that the RF power generator is altered in response to the feedback signal.

In a related embodiment, the bubble detector further compares the pre-treatment image and at least one intra-operative image. In an alternative embodiment, the bubble detector includes at least one of: a passive cavitation detector, a microphone, and a stethoscope. For example, the bubble detector determines a variation in echogenicity, a variation in Doppler spectra in duplex imaging, and a non-linear detection scheme. Further, the non-linear detection scheme includes harmonic signals and sub-harmonic signals.

In a related embodiment, detection of a presence in the target area of at least one bubble initiates at least one event in a closed loop feedback system. For example, the event includes an alteration in RF power. For example, the alteration in RF power includes an alteration of power to at least one tip of the RF probe. Further, the event includes a temporary extinction of an RF power generator signal.

In an alternative or an additional embodiment, a user is notified of a detection of a presence in the target area of at least one bubble, and the user initiates at least one event in an open loop feedback system. For example, the event includes an alteration of RF power. Further, the alteration in RF power includes an alteration of power to at least one tip of the RF probe. Further, the event includes a temporary extinction of an RF power generator signal.
Figure 1 is a diagram showing an ultrasound scanner, a RF probe or electrode, and an RF power generator, with the ultrasound scanner providing feedback control to the RF power generator.

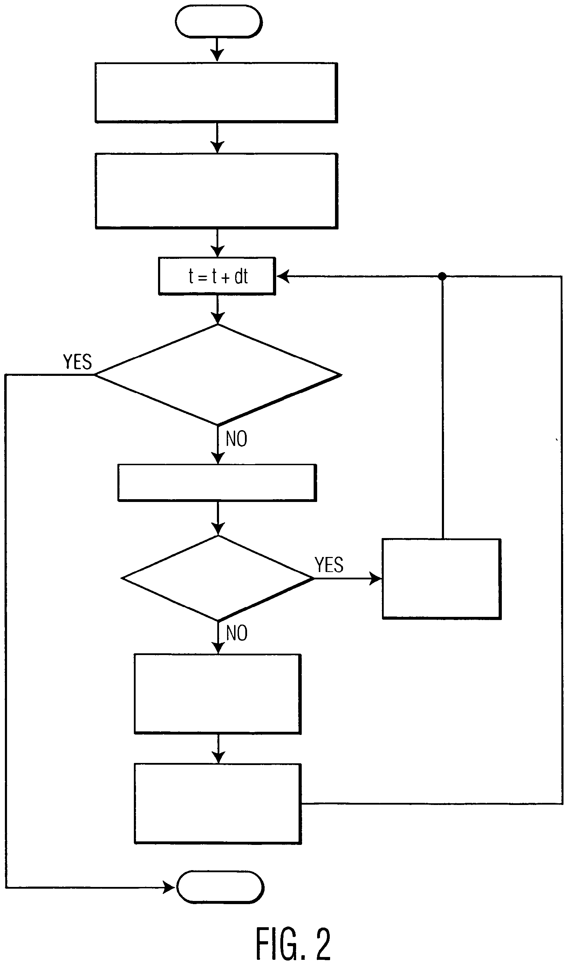

Figure 2 is a flowchart showing regulation of RF power using feedback received from ultrasound signals.

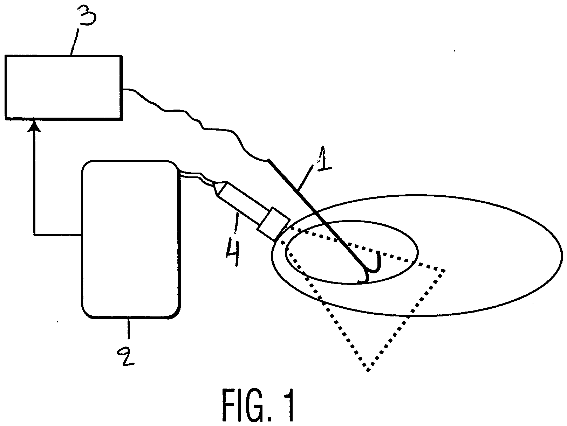

Ultrasound imaging for interventional guidance of RF ablation therapy has a wide variety of applications, including echocardiography, abdominal and breast imaging, and tumor ablation. An embodiment of the invention is shown in Figure 1. An ultrasound imaging system, e.g. an ultrasound scanner or ultrasound probe, is used to obtain an ultrasound image of a target area, for example an organ, a tissue, or a tumor. An RF probe, powered by an RF power generator, is inserted into the target area. The positioning of the RF probe can be guided using ultrasound images obtained by the ultrasound imaging system. The ultrasound imaging system also serves as a feedback control mechanism, relaying a feedback signal to the RF power generator, allowing power to the RF probe to be decreased or turned off if bubbles begin to form.

As shown in Figure 1, a tip of an RF probe 1 is inserted into a target area, e.g. an organ, a tissue, or a tumor, with the guidance of ultrasound to assure proper placement of the RF probe 1. An RF power generator 3 is turned on with preset parameters, and RF power is generated. The RF power generator 3 operates until an end signal is prompted. For example, if the RF power generator 3 has been operating for an amount of time (t) greater than a maximum amount of time (tₘₐₓ), the RF power generator 3 is automatically turned off. If tₘₐₓ has not been reached, then ultrasound images continue to be acquired. If bubbles are detected using ultrasound images, a feedback signal is generated which, for example, decreases or turns off the RF power automatically or a user can adjust the RF power manually in response to an alert or notification from the system. If no bubbles are detected, then a reading, for example a thermocouple reading or impedance reading, is obtained, and RF power can be adjusted based on the thermocouple or impedance parameters.

The invention includes an ultrasound imaging system 2 e.g. an ultrasound scanner or ultrasound probe. An ultrasound probe 4 is placed on the body of the patient. An ultrasound imaging system 2 shows an image the organ or tissue of interest through an ultrasound coupling gel. The ultrasound imaging system 2 is used initially to provide a pre-treatment image of a target area, e.g. an organ, tissue, or tumor, which is used for calibration. Continuous real-time acquisition of additional images is maintained by the ultrasound imaging system 2.

The ultrasound imaging system 2 can also be used for guiding insertion of the RF probe 1 into a target area, such as an organ, tissue, or tumor. The placement of the RF probe into an optimal location, the time of treatment and power deposition should be adequately controlled. Many factors are taken into account when choosing an optimal location for the RF probe 1. The size and localization of the tumor with respect to other anatomic structures are particularly important. In an exemplary case, the diameter of ablated volume is typically limited to about 2 to about 3 cm; multiple insertions are sometimes required to treat larger tumors. This requires treatment planning, and an imaging modality that allows guidance of needle insertion and that displays the extent of the ablated region.

The RF probe 1 includes a needle portion, which is inserted into the target area, e.g. an organ, a tissue, or a tumor. The RF probe 1 is usually inserted percutaneously, i.e. through the skin. During treatment, an adjuvant saline is infused at the tip of the RF probe 1. Ground pads are applied on another body surface of the patient, for example the thighs, prior to the RF power generator 3 being turned on.

The RF power generator 3 is turned on, causing heat to be generated in the tissue neighboring the RF probe tip by passage of RF current. RF electrodes are located at the tip of the RF probe, and allow RF power to be generated at the target area. An intra-operative image is produced from the continuous real-time acquisition. The pre-treatment image and the intra-operative image are compared to generate a feedback signal. The feedback signal is relayed to the RF power generator 3, and RF power is altered in response to the feedback signal to improve quality of the intra-operative image.

The ultrasound imaging system 2 is equipped with a bubble detector, which allows the presence of bubbles to be detected throughout the RF ablation procedure, and produces a feedback signal. The bubble detector compares the pre-treatment image and an intra-operative image. The bubble detector can also include or be associated with, for example, a passive cavitation detector, a microphone, or a stethoscope. The detection scheme of the bubble detector can be based on acquired scattered ultrasound waves, and can also rely on different types of acoustic features, including but not limited to sudden variation of echogenicity (e.g. in the image, or in a region of interest around a RF probe trip), variation in the Doppler spectra in duplex imaging, and non-linear detection schemes developed for microbubble contrast, such as the detection of strong harmonic and/or sub-harmonic signals.

A comparison between the pre-treatment image and an intra-operative image occurs in response to an index that determines the presence of bubbles in the target area. The index is derived from an ultrasonic image that indicates the presence of bubbles. As bubbles often appear as highly echogenic pockets, a decision can be made on the examination of several ultrasound features. If no bubbles are.detected, comparison between the pre-treatment image and an intra-operative image prompts a thermocouple reading or an impedance reading to be obtained.

Detection of the presence of bubbles in the target area initiates an event in a closed loop feedback system. When the index is higher than a certain threshold, a feedback signal is automatically sent to the RF generator 3. In response, there will be a decrease or a temporary extinction of the RF power generator signal, or an adjustment of power to other sections, tips or prongs of the RF probe 1. Alternatively, a user can initiate the alterations in RF power in an open loop feedback system.

The feedback generated by the system avoids increased heating and therefore limits boiling. As it is known that cell necrosis is triggered at temperatures lower than the boiling point, and that cell sensitivity to thermal treatments can also be increased by adjuvant therapy, e.g. chemotherapy or saline injection, it is expected that the extent of the coagulated volume should not be reduced even when preventing the occurrence of bubbles in the ultrasound imaging field.

It will furthermore be apparent that other and further forms of the invention, and embodiments other than the specific and exemplary embodiments described above, may be devised without departing from the scope of the appended claims , and therefore the description is intended to be exemplary and should not be construed as further limiting.

## Claims

1. A system comprising:
an ultrasound scanner (2), wherein the ultrasound scanner (2) is configured to acquire (i) a pre-treatment image of a target area for calibration, and (ii) at least one additional image of the target area for use during a radiofrequency (RF) ablation therapy;
a radiofrequency (RF) probe (1), wherein the RF probe (1) is configured to be inserted into the target area and to generate an RF current to heat the target area near a tip of the RF probe;
an RF power generator (3) configured to power the RF probe (1), and
a bubble detector, wherein the bubble detector is configured to indicate a presence of at least one bubble in the target area as a function of (a) a comparison of the pre-treatment image and at least one intra-operative image and (b) an index that determines a presence in the target area of at least one bubble, and responsive to an indication of the presence of at least one buble, automatically produce a feedback signal, wherein the RF power generator (3) is configured to be altered in response to the feedback signal to avoid heat-induced cavitation and to improve a quality of imaging during the RF ablation therapy, wherein altering includes (i) an alteration of power to at least one tip of the RF probe (1) or (ii) a temporary extinction of an RF power generator signal, and wherein responsive to no bubbles being detected, the bubble detector is configured for prompting an obtaining of a thermocouple reading or an impedance reading and adjusting RF power in response to thermocouple or impedance parameters.

2. The system according to claim 1, wherein the bubble detector further comprises at least one selected from the group consisting of: a passive cavitation detector, a microphone, and a stethoscope.

3. The system according the claim 1, wherein the bubble detector is further configured to determine a variation in echogenicity, a variation in Doppler spectra in duplex imaging, and a non-linear detection scheme.

4. The system according to claim 3, wherein the non-linear detection scheme comprises harmonic signals and sub-harmonic signals.

5. The system according to claim 1, wherein detection of a presence in the target area of at least one bubble initiates at least one event in a closed loop feedback system.

6. The system according to claim 1, wherein a user can be notified of a detection of a presence in the target area of at least one bubble, and wherein the user can initiate at least one event in an open loop feedback system.

## Patentansprüche

1. System, das Folgendes:
einen Ultraschall-Scanner (2), wobei der Ultraschall-Scanner (2) konfiguriert ist, um (i) ein Prä-Behandlungsbild eines Zielbereichs für die Kalibrierung und (ii) mindestens ein weiteres Bild des Zielbereichs zur Verwendung während einer Hochfrequenz-Ablationstherapie zu erfassen;
eine Hochfrequenz-Sonde (1), wobei die HF-Sonde (1) konfiguriert ist, um in den Zielbereich eingeführt zu werden und einen HF-Strom zu erzeugen, um den Zielbereich nahe einer Spitze der HF-Sonde zu erwärmen;
einen HF-Stromgenerator (3), der konfiguriert ist, um die HF-Sonde (1) mit Energie zu versorgen; und
einen Blasendetektor, wobei der Blasendetektor konfiguriert ist, um eine Anwesenheit von mindestens einer Blase in dem Zielbereich anzugeben als eine Funktion von (a) einem Vergleich des Prä-Behandlungsbildes und mindestens eines intraoperativen Bildes und (b) einem Index, der eine Anwesenheit von mindestens einer Blase in dem Zielbereich bestimmt, und um in Reaktion auf einen Hinweis auf die Anwesenheit von mindestens einer Blase automatisch ein Rückkopplungssignal zu erzeugen, wobei der HF-Stromgenerator (3) konfiguriert ist, um in Reaktion auf das Rückkopplungssignal verändert zu werden, um eine wärme-induzierte Kavitation zu vermeiden und die Bildgebungsqualität während der HF-Ablationstherapie zu verbessern, wobei das Verändern (i) eine Veränderung der Energie für die mindestens eine Spitze der HF-Sonde (1) oder (ii) eine vorübergehende Extinktion eines HF-Stromgeneratorsignals umfasst, und wobei in Reaktion darauf, dass keine Blasen detektiert werden, der Blasendetektor konfiguriert ist, um den Messwert eines Thermoelements oder einen Impedanzmesswert anzufordern und die HF-Energie in Reaktion auf die Thermoelement- oder Impedanzparameter anzupassen.

2. System nach Anspruch 1, wobei der Blasendetektor weiterhin mindestens ein Element aus der Gruppe bestehend aus einem passiven Kavitationsdetektor, einem Mikrofon und einem Stethoskop umfasst.

3. System nach Anspruch 1, wobei der Blasendetektor weiterhin konfiguriert ist, um eine Variation in der Echogenität, eine Variation in Doppler-Spektren bei Duplex-Bildgebung und ein nicht-lineares Detektionsschema zu ermitteln.

4. System nach Anspruch 3, wobei das nicht-lineare Detektionsschema harmonische Signale und sub-harmonische Signale umfasst.

5. System nach Anspruch 1, wobei die Detektion einer Anwesenheit von mindestens einer Blase in dem Zielbereich mindestens ein Ereignis in einem geschlossenen Rückkopplungssystem initiiert.

6. System nach Anspruch 1, wobei ein Benutzer über eine Detektion der Anwesenheit von mindestens einer Blase in dem Zielbereich benachrichtigt werden kann, und wobei der Benutzer mindestens ein Ereignis in einem offenen Rückkopplungssystem initiieren kann.

## Revendications

1. Système comprenant :
un échographe (2), ledit échographe (2) étant conçu pour acquérir (i) une image avant traitement d'une zone cible à des fins d'étalonnage et (ii) au moins une image supplémentaire de la zone cible à utiliser lors d'un traitement d'ablation par radiofréquence (RF) ;
une sonde de radiofréquence (RF) (1), ladite sonde RF (1) étant conçue pour être introduite dans la zone cible et pour produire un courant RF destiné à chauffer la zone cible à proximité de la pointe de la sonde RF ;
un générateur de puissance RF (3) conçu pour alimenter la sonde RF (1) ; et
un détecteur de bulles, le détecteur de bulles étant conçu pour indiquer la présence d'au moins une bulle dans la zone cible en fonction (a) d'une comparaison de l'image avant traitement et d'au moins une image intraopérative et (b) d'un indice qui détermine la présence dans la zone cible d'au moins une bulle, et en réponse à une indication de la présence d'au moins une bulle, pour produire automatiquement un signal de rétroaction, le générateur de puissance RF (3) étant conçu pour subir une modification en réponse au signal de rétroaction pour éviter la cavitation induite par la chaleur et pour améliorer la qualité de l'imagerie pendant le traitement d'ablation par RF, ladite modification comprenant (i) une modification de la puissance délivrée au moins à une extrémité de la sonde RF (1) ou (ii) une extinction temporaire d'un signal du générateur de puissance RF, et si aucune bulle n'est détectée, le détecteur de bulle étant conçu pour solliciter l'obtention d'une lecture de thermocouple ou d'une lecture d'impédance et pour régler la puissance RF en réponse à des paramètres de thermocouple ou d'impédance.

2. Système selon la revendication 1, dans lequel le détecteur de bulles comprend en outre au moins un élément choisi dans le groupe constitué des éléments suivants : un détecteur de cavitation passif, un microphone et un stéthoscope.

3. Système selon la revendication 1, dans lequel le détecteur de bulles est conçu en outre pour déterminer une variation d'échogénicité, une variation des spectres Doppler d'imagerie duplex, et un motif de détection non linéaire.

4. Système selon la revendication 3, dans lequel le motif de détection non linéaire comprend des signaux harmoniques et des signaux subharmoniques.

5. Système selon la revendication 1, dans lequel la détection de la présence dans la zone cible d'au moins une bulle provoque le lancement d'au moins un événement dans un système de rétroaction en boucle fermée.

6. Système selon la revendication 1, dans lequel un utilisateur peut être prévenu de la détection de la présence dans la zone cible d'au moins une bulle, et dans lequel l'utilisateur peut lancer au moins un événement dans un système de rétroaction en boucle fermée.
